(19) Europäisches Patentamt
European Patent Office
Office européen des brevets

(11) EP 3 325 457 B1

(12) EUROPEAN PATENT SPECIFICATION

(45) Date of publication and mention of the grant of the patent:
**01.01.2020 Bulletin 2020/01**

(21) Application number: **16733103.2**

(22) Date of filing: **01.07.2016**

(51) Int Cl.:
***C07D 307/46*** *(2006.01)*

(86) International application number:
**PCT/EP2016/065494**

(87) International publication number:
**WO 2017/012842 (26.01.2017 Gazette 2017/04)**

# (54) PROCESS FOR PREPARING FURAN-2,5-DICARBOXYLIC ACID

VERFAHREN ZUR HERSTELLUNG VON FURAN-2,5-DICARBOXYLSÄURE

PROCÉDÉ DE PRÉPARATION D'ACIDE FURANNE-2,5-DICARBOXYLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.07.2015 EP 15177884**

(43) Date of publication of application:
**30.05.2018 Bulletin 2018/22**

(73) Proprietor: **BASF SE**
**67056 Ludwigshafen am Rhein (DE)**

(72) Inventors:
- **GORDILLO, Alvaro**
**69126 Heidelberg (DE)**
- **WERHAN, Holger**
**69231 Rauenberg (DE)**
- **DEHN, Richard**
**67063 Ludwigshafen (DE)**
- **BLANK, Benoit**
**68535 Edingen-Neckarhausen (DE)**
- **TELES, Joaquim Henrique**
**67165 Waldsee (DE)**
- **SCHUNK, Stephan A.**
**69126 Heidelberg-Rohrbach (DE)**
- **PIEPENBRINK, Markus**
**48291 Telgte (DE)**
- **BACKES, René**
**68623 Lampertheim (DE)**
- **ZHANG, Lei**
**3453 NT De Meern (NL)**

(56) References cited:
**WO-A1-2012/017052 WO-A2-2013/033081**

- **HICHAM AIT RASS ET AL: "Selective aqueous phase oxidation of 5-hydroxymethylfurfural to 2,5-furandicarboxylic acid over Pt/C catalysts: influence of the base and effect of bismuth promotion", GREEN CHEMISTRY, vol. 15, no. 8, 1 January 2013 (2013-01-01), page 2240, XP055214536, ISSN: 1463-9262, DOI: 10.1039/c3gc40727f**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 325 457 B1

## Description

The present invention relates to a process for preparing furan-2,5-dicarboxylic acid (FDCA) (compound of the formula (I)) and to a corresponding use of a catalyst.

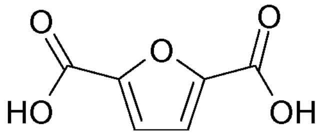

(I)

Further aspects of the present invention and the preferred configurations thereof are apparent from the description which follows, the working examples and the appended claims.

FDCA is an important compound for production of various products, for example surfactants, polymers and resins.

With increasing depletion of fossil feedstocks, starting materials based on renewable resources are needed, e.g. as alternatives to terephtalic acid (a compound used in the production of polyethylene terephtalate, PET). PET is based on ethylene and p-xylene which are usually obtained starting from of oil, natural gas or coal, i.e. from fossil fuels. While bio-based routes to ethylene (e.g. dehydration of bio-ethanol) are operated on commercial scale a straightforward access to bio-terephthalic acid remains difficult. FDCA is the best bio-based alternative to terephthalic acid (for further information see: Lichtenthaler, F.W., "Carbohydrates as Organic Raw Materials" in Ullmann's Encyclopedia of Industrial Chemistry, Wiley-VCH Verlag GmbH & Co. KGaA, Weinheim, 2010).

FDCA can be co-polymerized with mono-ethylene glycol to give polyethylene furanoate (PEF), a polyester with properties similar to PET.

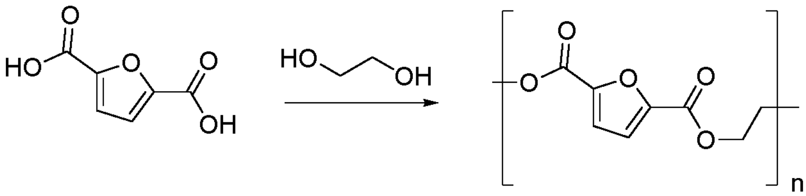

FDCA , polyethylene furanoate, PEF

FDCA is usually obtained starting from fructose and/or other hexoses via a catalyzed, preferably acid-catalyzed, dehydration to key intermediate 5-(hydroxymethyl)furfural (HMF) followed by oxidation to FDCA.

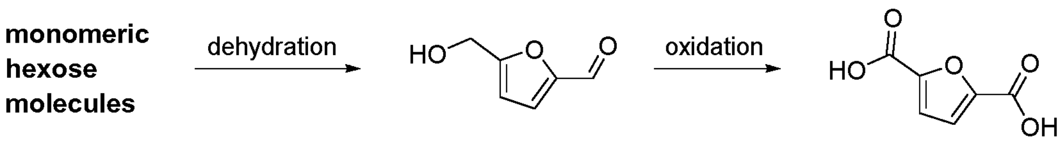


HMF FDCA

In the dehydration step by-products are formed, depending on the specific design of the process. A typical by-product is 5,5'-[oxy-bis(methylene)]bis-2-furfural (di-HMF) (V, see below).

In a typical process of preparing FDCA, a starting mixture comprising 5-(hydroxymethyl)furfural (HMF) is prepared by subjecting a material mixture, comprising one, two or more compounds selected from the group consisting of hexoses (monomeric hexose molecules, e.g. fructose), oligosaccharides comprising hexose units, and polysaccharides comprising hexose units, to reaction conditions so that a mixture comprising HMF, water and by-products (e.g. di-HMF) results. Under the reaction conditions oligo- and/or polysaccharides are usually depolymerised, and subsequently the resulting monosaccharides, e.g. monomeric hexose molecules, are converted into HMF. Hexoses, oligosaccharides and polysaccharides are typically selected from the group consisting of fructose, glucose, and cellulose.

During depolymerisation oligo- or polysaccharides are usually converted into monomeric hexose molecules by hydrolytic cleavage of the ether bonds connecting the different hexose units in an oligo- or polysaccharide molecule (e.g. cellulose). The products of a typical depolymerization process (monomeric hexose molecules) are present in their

aldehyde form.

Typically, according to routines at least in part previously undisclosed, depolymerization is conducted by using a catalyst, preferably in a one-pot-procedure. Typically a hydrophilic solvent is used (in particular water), e.g. in order to increase the amount of dissolved cellulose thus increasing the yield per process run. It is typically advantageous to conduct the conversion of cellulose into HMF by means of a heterogeneous catalyst in order to facilitate post-synthetic workup. In a typical depolymerization process, an aqueous solution is used as a solvent, sometimes comprising 50 wt.-% of water or more, based on the total weight of the depolymerization mixture used.

Alternatively, if monosaccharides are used as a starting material for preparing a starting mixture comprising HMF, water, and by-products, e.g. di-HMF, no depolymerisation step is needed.

Monosaccharides produced or provided are typically subjected to a dehydration process, wherein the aldehyde form of monomeric hexose molecules is typically transferred by isomerization (via e.g. ketone-enone tautomerization) into its ketone form which is subsequently converted into its ring form. After ring closure, the formed ring-closed hexose molecules are typically dehydrated (and optionally further isomerized) resulting in a mixture comprising HMF, by-products (e.g. di-HMF) and water, which can be used as a basic mixture in a process for preparing FDCA (preferably in a purified form).

Due to the insolubility of specific monomeric hexose molecules (e.g. fructose) in common organic solvents, the dehydration process step is usually performed in an aqueous environment so that an aqueous solution comprising HMF, by-products (e.g. di-HMF) and water is obtained as a (crude) mixture.

Isolation of HMF from such mixtures is challenging since HMF often undergoes side-reactions, e.g. (further) etherification to di-HMF. This is usually the case when water is removed during work-up (see for example US 2994645). Since two HMF-molecules are etherified the amount of by-products produced is correspondingly high.

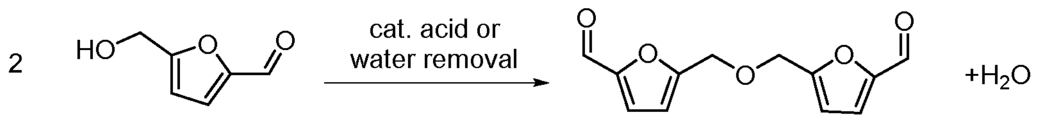


HMF di-HMF

Hence, the (crude) mixture comprising HMF and water is usually contaminated with by-products, in particular di-HMF, to a certain degree, as separation of HMF from the by-products, in particular di-HMF, is not possible with justifiable effort.

Common by-products (e.g. by-products as described above) are for example fructose in its ring form (RFF) (compound of the formula (III)), partially dehydrated fructose in its ring form (de-RFF) (compound of the formula (IV)), and 5,5'-[oxy-bis(methylene)]bis-2-furfural (di-HMF) (compound of the formula (V)). HMF (compound of the formula (II)) and di-HMF can be obtained in significant amounts from biomass, especially from biomass comprising hexoses and/or oligo- and/or polysaccharides as described above.

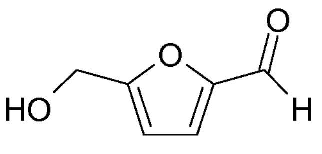

(II)

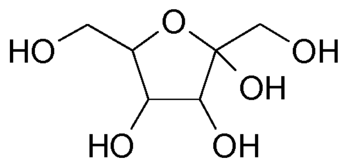

(III)

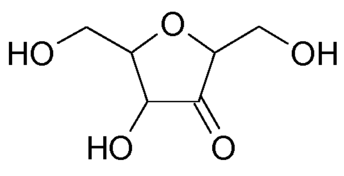

(IV)

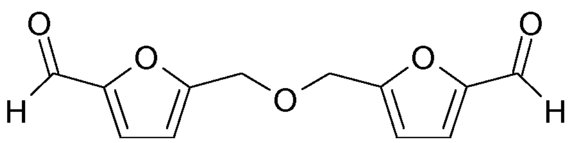

(V)

Different teachings regarding the isolation or preparation of FDCA have been reported in the patent literature:

WO 2008/054804 A2 relates to "Hydroxymethyl furfural oxidation methods" (title). It is disclosed that a reaction mixture having a mild basic pH can be provided by addition of sodium carbonate, the salts of FDCA having a distinctly elevated solubility in said reaction mixture compared to reaction mixtures having a neutral or acidic pH (cf. paragraph [0049]).

WO 2008/054804 A2 additionally discloses that twice as high a solubility of FDCA in an acetic acid/water mixture (volume ratio 40:60) is achieved, compared to the solubility in pure water (cf. paragraph [0058]).

WO 2013/033081 A2 discloses a "process for producing both by-based succinic acid and 2,5-furane dicarboxylic acid" (title). In example 46 and 47 a mixture of HMF and di-HMF (molar ratio HMF : di-HMF is 1:10) is converted to FDCA at 100°C.

US 2008/103318 discloses "hydroxymethyl furfural oxidation methods" (title) comprising the step of "providing a starting material which includes HMF in a solvent comprising water into reactor". The starting material is brought into contact "with the catalyst comprising Pt on the support material where the contacting is conducted at a reaction temperature of from about 50°C to about 200°C".

WO 2012/017052 A1 discloses a "process for the synthesis of 2,5-furandicarboxylic acid" (title).

Hicham Ait Rass et al. disclose a "selective aqueous phase oxidation of 5-hydroxymethyl furfural to 2,5-furandicarboxylic acid over Pt/C catalysts" (see titel of article in GREEN CHEMISTRY, vol. 15, no. 8,1 January 2013, page 2240).

US 2994645 discloses the "purification of hydroxymethyl furfural" (title). A process is disclosed wherein "gases and water by heating under a high vacuum" are initially removed.

The solubility of FDCA in aqueous solutions can be increased by addition of solubilizers. EP 0 356 703 A2 relates to a process for oxidizing 5-hydroxymethylfurfural (HMF) and discloses that the precipitation of reaction products during the oxidation of 5-hydroxymethylfurfural can be avoided, especially at relatively high concentrations, when a solubilizer which is inert with respect to the reaction participants under the selected reaction conditions is added to the reaction mixture. EP 0 356 703 A2 additionally discloses that suitable solubilizers are, for example, glycol ethers lacking free OH groups, especially dimethyl glycol ether, diethyl glycol ether and methyl ethyl glycol ether.

Very frequently, precipitation of FDCA leads to deactivation of the heterogeneous catalyst. WO 2013/191944 A1 discloses that, because of the very low solubility of FDCA in water, the oxidation of HMF has to be conducted in very dilute solutions, in order to avoid precipitation of the FDCA on the catalyst surface, since the process otherwise can no longer be conducted economically (cf. page 3).

Own observations show that the precipitation of FDCA on the internal and/or external catalyst surface of a heterogeneous catalyst can lead to contamination and possible deactivation of the heterogeneous catalyst. This involves coverage or coating of the catalytically active constituents of the heterogeneous catalyst by the precipitated FDCA, such that the catalytic constituents no longer come into contact with the reactants. The effect of such a contamination of the catalyst is that the catalyst does not display the same initial activity, if at all, and has to be replaced by new catalyst material which increases the costs. Especially in the case of utilization of costly catalysts, for example platinum catalysts, such a course of action is frequently uneconomic.

The aforementioned disclosure regarding the depolymerization or dehydration step also apply to (i) a process for preparing FDCA and (ii) a use of a catalyst according to the present invention as described in detail hereinbelow. In particular, the dehydration step or the successive steps of depolymerization and dehydration can be used to prepare a starting mixture as employed according to the present invention.

Despite the considerable efforts made by industry, there remains a need to provide an improved process for preparing FDCA from a starting mixture comprising HMF, di-HMF and water, which avoids or at least alleviates the disadvantages of the processes known to date and which can be operated in an economically advantageous manner. The process to be specified should favourably

- allow to reduce the complexity of reactor set-ups known in the prior art,
- allow to use a catalyst which can readily be separated from the product mixture after the reaction.

According to the invention, this object is achieved by a process for preparing furane-2,5-dicarboxylic acid, comprising the following step:

(a) preparing or providing a starting mixture comprising

5-(hydroxymethyl)furfural (HMF),

5,5'-[oxy-bis(methylene)]bis-2-furfural (di-HMF), and

water,

wherein the total amount of water in the starting mixture is at least 50 wt.-% based on the total weight of the starting mixture and

wherein the pH of the starting mixture is in the range of from 4.0 to 7.0,

(b) subjecting said starting mixture to oxidation conditions in the presence of an oxygen-containing gas and a catalytically effective amount of a heterogeneous catalyst comprising one or more noble metals on a support so that both HMF and di-HMF react to give furane-2,5-dicarboxylic acid in a product mixture also comprising water.

The "heterogeneous catalyst" preferably is a substance which is not soluble in water and/or is present in solid form.

The expression "both HMF and di-HMF react to give furane-2,5-dicarboxylic acid" indicates that under the oxidation conditions of step (b) HMF reacts and di-HMF reacts, and a first portion of the resulting furane-2,5-dicarboxylic acid is a product of HMF and a second portion of the resulting furane-2,5-dicarboxylic acid is a product of di-HMF.

The product mixture may also contain oxidation by-products. A non-limiting selection of oxidation by-products, which can be formed under oxidation conditions in step (b) of the process of the present invention, are 2,5-diformylfuran (DFF), 5-hydroxymethylfuran-2-carboxylic acid (HMFCA), 5-formylfuran-2-carboxylic acid (FFCA).

An "oxygen-containing gas" is a gas comprising gaseous compounds having one or more oxygen atoms per molecule. A preferred gaseous compound having one or more oxygen atoms per molecule is molecular oxygen ($O_2$).

Air is a preferred oxygen-containing gas.

The term "oxidation conditions" indicates conditions suitable for causing both HMF and di-HMF to react and to give furane-2,5-dicarboxylic acid in said product mixture also comprising water.

The oxygen-containing gas acts as an oxidizing agent.

Various types of reaction vessels can be used in step (b) to conduct the reaction of both HMF and di-HMF to furan-2,5-dicarboxylic acid (FDCA). In many cases an autoclave is used to conduct the reaction of HMF an di-HMF to FDCA. In many cases the reaction of HMF and di-HMF to FDCA is conducted in a batch reactor or in a semi-batch reactor. In other cases a plug flow or a fixed bed reactor is used.

As described above, in typical processes of the prior art, the reaction of two HMF molecules to one dimeric molecule (di-HMF) results in a high content of by-products and therefore in a low yield of FDCA. In contrast, the process according to the present invention converts both HMF and di-HMF into valuable FDCA, and thus the overall yield of the industrially important production of FDCA from hexoses is increased. In contrast to the teaching of WO 2013/033081 A2, a heterogeneous catalyst is used in the process of the present invention, thus allowing for a simplified work-up and other treatments of the product mixture and its ingredients.

Moreover, HMF and di-HMF are highly soluble in water thus increasing the maximum achievable starting concentration of HMF and di-HMF and therewith optimizing the space-time-yield of FDCA. Additionally, water is relatively inert under the oxidation conditions of the present invention as it cannot be oxidized as easily as other solvents (e.g. acetic acid). Thus, the oxygen-containing gas employed as oxidizing agent is used in a more efficient way.

Surprisingly, it has been found that the presence of HMF in the starting mixture is favourable when di-HMF is subjected to oxidation conditions in the presence of an oxygen-containing gas and a catalytically effective amount of a heterogeneous catalyst comprising one or more noble metals on a support, and is thereby converted into FDCA. Without wishing to be bound by any theory, it is presently believed that the conversion of the initially present HMF into FDCA proceeds in a shorter time frame in comparison with the conversion of di-HMF to FDCA. Upon conversion of the initially present HMF into FDCA the pH of the reaction mixture decreases, as the reaction product FDCA is a dicarboxylic acid. The increasing concentration of protons in the reaction mixture catalyzes the hydrolytic cleavage of di-HMF into two HMF molecules thus increasing the concentration of HMF. In turn, the HMF formed by cleaving di-HMF is subsequently quickly converted into FDCA thereby further decreasing the pH and increasing the rate of the cleaving reaction. This allows to produce FDCA from di-HMF in an economically valuable time frame with no need of additional agents as used according to the prior art, for example HBr (see example 46 and 47 in WO 2013/033081) or similarly corrosive agents. Hence, at the beginning of the reaction the concentration of HMF should be sufficiently high to initiate the conversion of di-HMF to FDCA (in contrast to WO2013/033081). The deliberate presence of HMF for the acceleration of a process for preparing FDCA from di-HMF is therefore a primary reason for the advantages provided by the present invention.

According to the present invention, the oxidation of HMF and di-HMF into FDCA is conducted in a starting mixture comprising water. Preferably, in the starting mixture of step a) the total amount by weight of di-HMF, preferably resulting from a previous process step (e.g. process step (a2) as described hereinbelow), and HMF is higher than the total amount of other organic compounds. The starting mixture used in the process according to the invention in step a) may comprise a comparatively high total concentration of reactant compound(s), HMF and di-HMF. This regularly leads to precipitation of FDCA during the catalytic conversion in step (b) and hence to the product mixture comprising FDCA in solid or dissolved form and the heterogeneous catalyst in solid form.

In the process according to the invention, in step (b), both the heterogeneous catalyst and FDCA can be present in solid form. However, preferably the heterogeneous catalyst is present in solid form and FDCA is present in its dissolved form. A heterogeneous catalyst used in step (b) may be part of a mixture of two, three or more than three heterogeneous catalysts. Typically, the product mixture formed in step (b) of a process according to the invention at least comprises water and a heterogeneous catalyst in separate phases, but many times comprises as a further solid phase the product FDCA. The proportion of the dissolved FDCA in the aqueous phase is typically low, because of the low solubility product of FDCA in water or aqueous solutions. Preferably, the aqueous phase of the product mixture produced in step (b) of the present invention is a saturated solution with respect to FDCA.

The product mixture obtained in step (b) can be optionally subjected to further treatment conditions resulting in a second product mixture.

WO 2013/191944 A1 discloses that, under pressure and at a temperature in the range of 120°C to 240°C, FDCA in solid form is dissolved in an appropriate aqueous solvent. At appropriate temperature and appropriate pressure, an overheated aqueous solution may comprise a total proportion of dissolved FDCA in the range of from 10 to 20% by weight, based on the total amount of the aqueous solution.

Heating under pressure of the product mixture of step (b), or of the second product mixture obtained by subjecting the product mixture of step (b) to further treatment conditions, each comprising both FDCA in solid or dissolved form and the heterogeneous catalyst in solid form, regularly dissolves at least some of the FDCA deposited on or within the pore-system of the heterogeneous catalyst (e.g. the pore-system of the support material). Preferably, a subsequent (further treatment) step comprises heating the heterogeneous catalyst as present at the end of step (b) or as present at the end of an intermediate step following step (b) so that the activity of the heterogeneous catalyst after heating (i.e. its capability to act as a catalyst for the oxidation of HMF to FDCA) is increased in comparison with the heterogeneous catalyst as present at the end of step (b).

More preferably, the process of the present invention comprises a subsequent (further treatment) step as described above comprising heating the heterogeneous catalyst as present at the end of step (b) or as present at the end of an intermediate step following step (b) so that the activity of the heterogeneous catalyst after heating (i.e. its capability to act as a catalyst for the oxidation of HMF to FDCA) is increased, wherein the activity of the heterogeneous catalyst after the heating is increased by at least 5%, preferably by at least 10%, more preferably by at least 20%, even more preferably by at least 30%, most preferably by at least 50% in comparison with the activity of the heterogeneous catalyst as present at the end of step (b).

A process of the invention is preferred wherein the product mixture resulting in process step (b) is subjected to additional separation, purification and/or to (re-)crystallization steps to obtain purified FDCA.

In many cases a process of the invention is preferred, wherein

the starting mixture has a molar ratio of HMF to di-HMF in the range of from 100 to 0.8, preferably in the range of from 100 to 0.9

and/or

the total weight of HMF and di-HMF in the starting mixture is in the range of from 0.1 to 50 wt.-%, preferably in the range of from 1 to 30 wt.-%, more preferably in the range of from 1 to 20wt.-%, based on the total weight of the starting mixture.

In many preferred practical situations the starting mixture has a molar ratio of HMF to di-HMF in the range of from 100 to 20, in many other situations the range of from 10 to 0.9 is preferred.

In the starting mixture, these ranges of molar ratios of HMF and di-HMF and/or this range of the total weight of HMF and di-HMF are preferred as those values represent optimum values for the production of FDCA from HMF or di-HMF. When working within these ranges, a relatively low amount of by-products is produced and the reaction can be conducted in an economically acceptable time frame.

A concentration of over 50 wt.-% of HMF and di-HMF, based on the total starting mixture is in many cases disadvantageous, as the solubility characteristic of the reaction mixture is changed so that FDCA produced will likely precipitate, thus complicating post-synthetic work-up.

By using water as a solvent in a process of the invention an environmentally friendly solvent is used. Moreover, the higher the content of water in the starting mixture the more HMF and di-HMF can be dissolved and thus the more FDCA can be produced per batch.

Preferred is a process of the present invention, wherein the pH of the starting mixture 4.5 or higher, more preferably 5.0 or higher, even more preferably 5.5 or higher, or preferably the pH of the starting mixture is in the range of from 4.5 to 7.0, more preferably the pH of the starting mixture is in the range of from 5.0 to 7.0, even more preferably the pH of the starting mixture is in the range of from 5.5 to 7.0.

The conversion of HMF into FDCA in a starting mixture is conducted with a pH of 4.0 or higher, as the produced FDCA is very well soluble in such a reaction mixture with a pH of 4.0 or higher. Starting mixtures with a pH below 4.0 are disadvantageous because a low pH in the starting mixture will result in a product mixture with a correspondingly low pH causing unfavourable precipitation of FDCA.

In the process of the present invention the addition of solubilizers is optional. Preferably, the starting mixture

in step (b) does not comprise a solubilizer for FDCA.

Preferably, in step (b) of the process of the present invention the development of the pH in the mixture subjected to oxidation conditions is not controlled by the addition of alkaline reagents.

A process of the present invention is preferred, wherein the total amount of HMF in the starting mixture is in the range of from 0.1 to 40 wt.-%, preferably in the range of from 1 to 30 wt.-%, based on the total weight of the starting mixture.

As mentioned above, FDCA produced from initially present HMF accelerates the hydrolytic cleavage of di-HMF and thus accelerates the overall reaction. Therefore, concentrations of HMF in the starting mixture below 0.1 are not advantageous. On the other hand is a concentration of over 40 wt.-% of HMF, based on the total amount of the starting mixture, disadvantageous as the solubility characteristic of the reaction mixture is changed so that FDCA produced will likely precipitate.

In particular, a process of the invention is preferred, wherein the total amount of di-HMF in the starting mixture is in the range of from 0.1 to 40 wt.-%, preferably in the range of from 0.1 to 30 wt.-%, more preferably in the range of from 0.1 to 10 wt.-%, even more preferably in the range of from 0.2 to 6 wt.-%, based on the total weight of the starting mixture.

A concentration of over 40 wt.-% of di-HMF, based on the total weight of the starting mixture, is disadvantageous as the solubility characteristic of the reaction mixture is changed so that the FDCA produced will likely precipitate.

Preferred is a process of the invention, wherein the pH of the product mixture is below 7 and wherein preferably the pH of the product mixture is in the range of from 1 to 4.

According to the present invention the pH of the reaction mixture can be monitored in order to correspondingly monitor the conversion to FDCA during the reaction process. It is preferred to have a product mixture with a pH below 7 (preferably below 4) which generally means that an economically valuable amount of HMF or di-HMF to FDCA was converted.

A process of the invention is preferred, wherein said starting mixture at a temperature in the range of from 70°C to 200°C, preferably in the range of from 80°C to 180°C, more preferably in the range of from 90°C to 170°C, even more preferably in the range of from 100°C to 140°C, is subjected to said oxidation conditions in the presence of said oxygen-containing gas and said catalytically effective amount of a heterogeneous catalyst comprising one or more noble metals on a support, so that both HMF and di-HMF react to give furane-2,5-dicarboxylic acid in the product mixture also comprising water and oxidation by-products.

On the one hand, lower reaction temperatures typically result in a reduced reaction rate thus significantly increasing the time needed for the oxidation of HMF or di-HMF to FDCA and making the process economically inefficient.

On the other hand, too high temperatures can lead to overoxidation, a too high reaction rate, an increased production of oxidation by-products and hardly controllable reaction conditions which require costly safety measures.

In many cases, a process is preferred as described above (or as preferably described above), wherein said starting mixture is subjected to said oxidation conditions in a pressurized reactor, wherein during said reaction of HMF and di-HMF to FDCA oxygen or an oxygen-containing gas is continuously (or optionally and less preferred discontinuously) fed into and simultaneously removed from said reactor

In some cases the pressure at which the reaction is conducted, depends on the headspace volume of the reactor used which has to accommodate at least the required stoichiometric amount of oxygen-containing gas to fully convert the reactants HMF and di-HMF. A high pressure (of, for example, 20 or, for example, even 100 bar) is required in cases, where no continuous or discontinuously feed of an oxygen-containing gas is used, e.g. in a case where the reactor is once pressurized with an at least stoichiometric amount of an oxygen-containing gas at the beginning of the reaction without further manipulation of the pressure in the reactor.

In other cases consumed oxygen-containing gas is continuously or discontinuously replaced by fresh oxygen-containing gas. In such cases an oxygen partial pressure in the range of from 200 mbar and 10 bar is preferred.

A process of the present invention is preferred, wherein said starting mixture is subjected to said oxidation conditions in a pressurized reactor, wherein the oxygen partial pressure in the reactor at least temporarily is in the range of from 100 mbar to 20 bar, preferably in the range of from 200 mbar to 10 bar, during the reaction of both HMF and di-HMF to furane-2,5-dicarboxylic acid.

A skilled person will choose suitable oxidation conditions according to his specific needs. In many cases, the oxidation is conducted at a pressure of 1 to 100 bar, preferably at a pressure of 1 to 20 bar in an atmosphere of an oxygen-containing gas or a mixture of an oxygen-containing gas and another gas (which is preferably inert under the reaction conditions).

Working under a pressure below 1 bar is not preferred as it requires additional technical measures thus increasing the complexity of the reaction system. In order to work at pressures above 20 bar additional safety equipment is necessary in order to fulfil specific safety requirements.

A process of the invention is preferred wherein said starting mixture does not comprise a catalytically effective amount of a homogeneous oxidation catalyst selected from the group of cobalt, manganese, and bromide compounds,

and mixtures thereof.

In order to separate a homogeneous oxidation catalyst from a reaction mixture technically complicated separation units are required in the overall product plant thus increasing material and energy costs. Thus, according to the present invention the presence of one or more homogeneous oxidation catalysts is not preferred.

More specifically, a process of the invention is preferred wherein the total amount of cobalt and manganese and bromide ions in the starting mixture is below 100 ppm, preferably below 20 ppm.

It is of particular interest to avoid toxic or corrosive compounds, in particular cobalt and manganese compounds as well as bromide compounds. The latter drastically increases the corrosiveness of the reaction mixture and therefore requires specially coated reactor vessels which incur high costs.

A process of the invention is preferred wherein the total amount of carboxylic acid ions and carboxylic acid in the starting mixture is below 10 wt.-%, preferably below 5 wt.-%.

Depending on the nature of the acid, e.g. the number of acid groups per molecule and its specific structure, the presence of a specific carboxylic acid or of its anions modifies the pH of the reaction mixture and therefore complicates the monitoring of the progress of the FDCA forming reactions by pH. This effect is even more pronounced as the carboxylic acids present can be oxidized by an oxygen-containing gas under the oxidation conditions of step (b) as described above to compounds with changed acidity, and this may effect the pH further. In such a case the pH could no longer be used as a measure for the progress of the FDCA forming reactions.

Moreover, the side reactions between carboxylic acids and the oxygen-containing gas results in an inefficient use of the oxygen-containing gas as an oxidizing agent for HMF and di-HMF.

A process of the present invention is preferred, wherein the total amount of acetate ions and acetic acid in said starting mixture is below 10 wt.-%, preferably below 1 wt.-%.

A process of the invention is preferred, wherein the step of preparing said starting mixture (according to step (a)) comprises

(a1) preparing or providing a material mixture comprising
one, two or more compounds selected from the group consisting of hexoses, oligosaccharides comprising hexose units, and polysaccharides comprising hexose units,

(a2) subjecting said material mixture to reaction conditions so that a mixture results comprising

HMF,

di-HMF, and

water,

(a3) optionally subjecting the mixture resulting from step (a2) to additional treatment conditions, preferably without adding a carboxylic acid and/or without adding an acidic solvent for dissolving HMF and di-HMF,

so that said starting mixture results.

The term "acidic solvent" designates an aqueous solvent mixture having a pH below 6 and/or a solvent (aqueous or non-aqueous) comprising a substance having a pKa below 5.

The process step of subjecting the mixture to reaction conditions so that a mixture results comprising HMF, di-HMF, and water (i.e., process step (a2) as defined above) often comprises a depolymerization and/or a dehydration step as described above. All aspects of a depolymerization and/or a dehydration step discussed herein above in the context of a process of preparing a starting mixture for a process for preparing furane-2,5-dicarboxylic acid apply *mutatis mutandis* for a process according to the present invention.

In some cases, it is advantageous to conduct depolymerization and dehydration step (step (a2) as defined above) by using the same catalyst and/or the same reaction mixture and/or the same reactor.

In particular, a step of preparing said starting mixture is preferred as described above (or as preferably described above) wherein process step (a3) is omitted (no additional treatment conditions are needed, e.g. solvent change) and the mixture resulting in process step (a2) is the starting mixture prepared in process step (a) and subjected to oxidation conditions of process step (b).

In some cases, it is advantageous to conduct depolymerization and dehydration step (step (a2)), and the oxidation of HMF and di-HMF to FDCA (step (b)) in the same reactor.

As described above, di-HMF is produced as a by-product during the conversion of hexoses or oligosaccharides or polysaccharides (e.g. cellulose) to HMF. It is therefore a further achievement of the present invention that di-HMF like HMF is converted to FDCA and thus contributes to an increase of the overall yield of the process. The addition of

acidic solvent and/or carboxylic acid should be avoided in order to allow for a monitoring of the process of the reaction by measuring the pH.

Another advantage of the process of the present invention as described above is the use of water as a solvent. According to the present invention it is preferred that after successful conversion of said one, two or more compounds selected from the group consisting of hexoses, oligosaccharides comprising hexose units, and polysaccharides comprising hexose units into HMF (and di-HMF) the aqueous material mixture obtained in step (a2) (or the aqueous material mixture obtained after additional treatment in step (a3)) is directly fed into the reactor where the produced HMF and di-HMF are converted into FDCA (according to step (b) of the present invention).

It is however even more advantageous if process steps (a2) and (b) are performed in the same reactor, with an intermediate step (a3) in the same reactor or without an intermediate step (a3). Therewith the need for complicated and costly solvent separation, solvent exchange or solvent purification between steps (a2) and (b) is reduced or prevented. In many cases, two heterogeneous catalysts are used in step (a2) and step (b). However, in some cases, the catalyst can be the same for both steps. Therefore, the overall process can be simplified by using the same solvent system throughout steps (a1) to (b).

In particular, a process of the invention is preferred, wherein in said heterogeneous catalyst comprising one or more noble metals on a support

(i) at least one of said noble metals is selected from the group consisting of gold, platinum, iridium, palladium, osmium, silver, rhodium and ruthenium,
and/or

(ii) said support is selected from the group consisting of carbon, metal oxides, metal halides, and metal carbides.

The specific noble metals as stated above under item (i) catalyze the reaction of HMF into FDCA. Suitable supports for immobilizing the noble metals as mentioned above are the supports stated above under item (ii) because they do not negatively affect the reaction kinetics during the conversion of di-HMF and HMF into FDCA.

A process of the invention is particularly preferred, wherein in said heterogeneous catalyst comprising one or more noble metals on a support
at least one of said noble metals is selected from the group consisting of platinum, iridium, palladium, osmium, rhodium and ruthenium, preferably platinum,
and
said support is carbon.

Carbon is a suitable support for immobilizing noble metals as described above, in particular platinum, as it does not negatively influence the reaction kinetics of the conversion of HMF and di-HMF into FDCA.

A process of the invention is preferred, wherein in said heterogeneous catalyst comprising one or more noble metals on a support

said one or one of said more noble metals is platinum and said support is carbon,
and

the content of platinum on the support is in the range of from 0.1 to 20 wt.-%, preferably 1 to 10 wt.-%, based on the total weight of the heterogeneous catalyst comprising one or more noble metals on a support.

In order to sufficiently accelerate the reaction of HMF and di-HMF into FDCA, the loading of platinum on the support should be at least 0.1 wt.-% (preferably at least 1 wt.-%), based on the total weight of heterogeneous catalysts comprising one or more noble metals on a support.

In contrast thereto if too much platinum is immobilized on a support the conversion per platinum atom decreases due to a lower average accessibility of the platinum atoms thus leading to a higher waste of noble metals and thus higher costs.

A process of the invention is preferred, wherein in said heterogeneous catalyst comprising one or more noble metals on a support the molar ratio of said one or one of said more noble metals to the total amount of HMF and di-HMF is in the range of from 1:1 000 000 to 1:10, preferably in the range of from 1:10 000 to 1:10, more preferably in the range of from 1:1 000 to 1:100, preferably said one or one of said more noble metals is platinum.

It is advantageous to convert as much HMF and di-HMF per noble metal atom as possible to FDCA to increase the yield of FDCA per batch and to efficiently use the precious noble metal.

A process of the present invention is preferred, wherein the process is not a process comprising all of the following steps:

A) in an aqueous reactant mixture, catalytically converting one or more organic reactant compounds by means of at least one heterogeneous catalyst, so as to form a first product suspension comprising furan-2,5-dicarboxylic acid in solid form and the heterogeneous catalyst in solid form,

B) heating under pressure

1. this first product suspension, or

2. a second product suspension prepared therefrom by further treatment, each comprising furan-2,5-dicarboxylic acid in solid form and the heterogeneous catalyst in solid form, such that furan-2,5-dicarboxylic acid dissolves fully or partly, resulting in a first aqueous product phase comprising dissolved furan-2,5-dicarboxylic acid, and then

C) separating the heterogeneous catalyst from this first aqueous product phase comprising dissolved furan-2,5-dicarboxylic acid, or from a second product phase which results therefrom through further treatment and comprising dissolved furan-2,5-dicarboxylic acid.

A process of the invention is preferred wherein the product mixture obtained in step (b) comprises FDCA in dissolved form, and wherein the product mixture obtained in step (b) preferably does not comprise FDCA in solid form.

As described above, the precipitation of FDCA in the presence of a heterogeneous catalyst is highly disadvantageous, as the effect of the precipitation of FDCA is that both heterogeneous catalyst and FDCA are present in solid form and can no longer be separated from one another in a simple manner. As described above, very frequently, precipitation of FDCA leads, incidentally, to deactivation of the heterogeneous catalyst. The precipitation of FDCA on the internal and/or external catalyst surface of a heterogeneous catalyst can lead to contamination and possible deactivation of the heterogeneous catalyst. This involves coverage or coating of the catalytically active constituents of the heterogeneous catalyst by the precipitated FDCA, such that the catalytic constituents no longer come into contact with the reactants. The effect of such a contamination of the catalyst is that the catalyst does not display the same initial activity, if at all, and has to be replaced by new catalyst material which increases the costs. Especially in the case of utilization of costly catalysts, for example platinum catalysts, such a course of action is frequently uneconomic.

The present invention also relates to the use of a catalyst comprising one or more noble metals on a support as an heterogeneous oxidation catalyst for accelerating in an aqueous starting mixture the conversion of both HMF and di-HMF to furane-2,5-dicarboxylic acid. Herein the catalyst preferably is a catalyst as defined hereinabove or in the attached claims. Preferred is the use of a catalyst comprising one or more noble metals (preferably gold, platinum, iridium, palladium, osmium, silver, rhodium and ruthenium) on a support (preferably carbon, metal oxides, metal halides, and metal carbides). Moreover, the use of a catalyst comprising one or more noble metals on a support as an heterogeneous oxidation catalyst allows to conduct steps (a1), (a2), optionally (a3), and (b) without solvent exchange and without addition of expensive chemicals like acetic acid.

Generally, all aspects of the present invention discussed herein above in the context of a process of preparing furane-2,5-dicarboxylic acid according to the present invention apply *mutatis mutandis* for the use of a catalyst according to the present invention. And likewise, all aspects of the inventive use of a catalyst discussed herein above or below apply *mutatis mutandis* for a process for preparing furane-2,5-dicarboxylic acid according to the present invention.

Preferred is the use of a catalyst according to the present invention in processes as described above, in particular in processes of making FDCA. All aspects of or associated with processes of the invention as described above (or as preferably described above) can also be conducted by or in combination with the use of a catalyst according to the invention.

By using a catalyst according to the present invention, it is possible to simultaneously convert di-HMF and HMF into valuable FDCA and thus to increase the overall yield of the industrial important production of FDCA from hexoses (e.g. fructose) etc.

Further advantages of a use of a catalyst according to the present invention are as described herein above generally in the context of the process of the present invention and more specifically with respect to preferred aspects of this process.

In many cases the use according to the invention of a catalyst is preferred, wherein the pH of the starting mixture is 4.0 or higher, preferably 4.5 or higher, more preferably the pH of the starting mixture is in the range of from 4.0 to 7.0, most preferably the pH of the starting mixture is in the range of from 4.5 to 7.0. The corresponding advantages are as discussed above.

It is thus an achievement of the present invention to allow for a use of catalyst as defined above which is active in the conversion of both HMF and di-HMF into FDCA and can readily be separated and subsequently reused.

Examples:

Catalyst screening experiments:

Catalyst screening was carried out in a series of single experiments designated "Experiment 1" to "Experiment 3". In each single experiment "1" to "3" the organic reactant compounds HMF and di-HMF were in parts catalytically converted by means of a heterogeneous platinum catalyst to FDCA. The general experimental procedure for each screening experiment of "1" to "3" was as follows:
In a first step, by filling into a steel autoclave reactor (inner volume 90 ml) specific amounts of deuterated water ($D_2O$, 99,9 atom%, Sigma Aldrich (151882)), HMF (99+%), and di-HMF (99+%) an aqueous starting material mixture was prepared having a composition similar to the composition of HMF feed-streams usually obtained in sugar dehydration). The amounts of the reactants and $D_2O$ are identified in table 1 below:

Table 1:

| | |
|---|---|
| $D_2O$ | 28.5 g |
| total amount of reactants HMF and di-HMF | 1.5 g |
| HMF. | 1.0, 0.75 or 0.5 g |
| di-HMF* | 0.5, 0.75 or 1.0 g |
| *di-HMF can, e.g., be synthesized according to WO 2013/033081, example 45. | |

The starting concentration $C_{0[HMF+di\text{-}HMF]}$ of HMF + di-HMF in each aqueous reactant mixture was correspondingly 5 % by weight, based on the total mass of the aqueous reactant mixture (total mass of deuterated water, HMF and di-HMF). The solid heterogeneous catalyst (0.928 g of 5wt%Pt/C, 50wt%$H_2O$) was added to the respective aqueous reactant mixture and, thus, a reaction mixture comprising deuterated water, HMF, di-HMF, and the heterogeneous catalyst was obtained.

In a second step, the filled reactor was tightly sealed and pressurized with synthetic air (total pressure 100 bar to obtain conditions so that both HMF and di-HMF react to give FDCA. The starting mixture in the reactor comprising HMF, di-HMF and deuterated water was heated to a temperature of 100°C while stirring at 2000 rpm. After reaching 100°C, this temperature was maintained for 18 hours while continuing stirring the heated and pressurized reaction mixture during the reaction time. A product mixture comprising FDCA, oxidation by-products, deuterated water and the heterogeneous catalyst resulted.

In a third step, after the temperature had been maintained for 18 hours, to give a cooled product mixture the steel autoclave reactor was

(i) allowed to cool down to room temperature (approximately 22 °C),

(ii) depressurized and

(iii) opened.

The product mixture obtained was in the form of a suspension.

For the purpose of product analysis of the cooled product mixture, a solution of deuterated sodium hydroxide (NaOD, 40 wt.-% in $D_2O$, 99.5 atom% D, Sigma Aldrich) was carefully added to the product mixture until a slightly alkaline product mixture having a pH in the range of from 9 to 10 was reached. The slightly alkaline product mixture comprised the disodium salt of FDCA in completely dissolved form, and the heterogeneous catalyst in solid form.

In a fourth step, the heterogeneous catalyst in the slightly alkaline product mixture was separated from the solution by syringe filtration, and the filtrate (i.e. the remaining solution comprising the disodium salt of FDCA in completely dissolved form) was subsequently analyzed by $^1$H-NMR spectroscopy. $^1$H-NMR spectroscopy was used to determine the concentration of FDCA, FFCA, HMF and di-HMF.

NMR analysis:

NMR sample preparation and NMR measurements:

3-(Trimethylsilyl)propionic-$d_4$ acid sodium salt (Standard 1, 68.39 mg, corresponding to 0.397 mmol, 98+ atom% D, Alfa Aesar (A14489)) and Tetramethylammonium iodide ($Me_4N+I-$, Standard 2, 80.62 mg, corresponding to 0.397 mmol, 99%, Alfa Aesar (A12811)) were added as internal standards to 5.0 g of a slightly alkaline product mixture, exhibiting a pH value in the range of from 9 to 10. Finally, 0.7 ml of this prepared sample liquid were transferred into a NMR tube for $^1$H NMR quantification experiments.

NMR-spectra were recorded in $D_2O$ at 299 K using a Bruker-DRX 500 spectrometer with a 5mm DUL 13-1H/19F Z-GRD Z564401/11 probe, measuring frequency 499.87 MHz. Recorded Data were processed with the software Topspin 2.1, Patchlevel 6 (Supplier: Bruker BioSpin GmbH, Silberstreifen 4, 76287 Rheinstetten, Germany).

Interpretation of NMR spectra:

Interpretation of NMR spectra is based on published reference data as indicated below.

Disodium salt of FDCA (disodium salt of compound of formula (I)):

$^1$H NMR (500 MHz, D2O, 299 K): 6.97 ppm (2H, s, furan-H); 13C{1H} NMR: 166.1 ppm (-COO), 150.0 ppm (furan C atoms), 115.8 ppm (furan C atoms).

Reference: J. Ma, Y. Pang, M. Wang, J. Xu, H. Ma and X. Nie, J. Mater. Chem., 2012, 22, 3457-3461.

Sodium salt of FFCA (sodium salt of compound of formula V):

$^1$H NMR (500 MHz, D2O, 299 K): 9.49 ppm (1H, s, -CHO); 7.42 ppm (1H, d, 3J= 3.67 Hz, furan-H); 7.03 ppm (1H, d, 3J= 3.67 Hz, furan-H).

Reference: A. J. Carpenter, D. J. Chadwick; Tetrahedron 1985, 41(18), 3803-3812.

Screening experiments:

In each single experiment a cooled product mixture, and based thereon a slightly alkaline product mixture comprising the disodium salt of FDCA in completely dissolved form was obtained. As shown in Table 1, HMF conversion in mol% and yield in mol% are summarized.

Table 1: Relevant parameters of catalyst screening experiments.

| Exp. | Catalyst [g] | HMF [g] | di-HMF [g] | HMF Conversion [mol%] | di-HMF Conversion [mol%] | $Y_{FDCA}$ [mol%] | $Y_{FFCA}$ [mol%] |
|---|---|---|---|---|---|---|---|
| 1 | 0.928 | 1.00 | 0.50 | 100 | 100 | 78.3 | <1.0 |
| 2 | 0.928 | 0.75 | 0.75 | 100 | 100 | 63.4 | 1.4 |
| 3 | 0.928 | 0.50 | 1.00 | 100 | 100 | 48.2 | 3.3 |

Table 2: Relevant parameters of catalyst screening experiments.

| Exp. | HMF [g] | HMF [mmol] | di-HMF [g] | di-HMF [mmol] | ratio of HMF: di-HMF | FDCA [mmol] | $Y_{FDCA}$ [mol%] | $Y_{FFCA}$ [mol%] | $C_{HMF+di-HMF}$ [mol%] | $Y_{min,di-HMF}$ [mol%] |
|---|---|---|---|---|---|---|---|---|---|---|
| 1 | 1.00 | 7.93 | 0.50 | 2.13 | 3.72 | 9.58 | 78.3 | <1.0 | 65.1 | 13.2 |
| 2 | 0.75 | 5.95 | 0.75 | 3.20 | 1.86 | 7.86 | 63.4 | 1.4 | 48.2 | 15.2 |
| 3 | 0.50 | 3.96 | 1.00 | 4.27 | 0.93 | 6.07 | 48.2 | 3.3 | 31.7 | 16.5 |

HMF conversion in mol% was calculated as follows (di-HMF conversion was calculated accordingly):

$$\text{HMF Conversion [mol\%]} = [1-(C_{final[HMF]}/C_{0[HMF]})]*100,$$

wherein $C_{[HMF]}$ is the concentration in % by weight measured in the slightly alkaline product mixture and $C_{0[HMF]}$ is the concentrations in % by weight measured based on the added amount of HMF and the volume of the starting mixture.

"Conversion [mol%]" and "yield [mol%]" are average values calculated from a first value based on internal standard 1 and a second value based on internal standard 2 (general deviation is less than 5 %).

The yield definition (exemplified for FDCA):

$$Y_{FDCA} = \frac{n_{FDCA}}{n_{HMF} + 2 \cdot n_{di\text{-}HMF}}$$

wherein

$$n_{[FDCA]} = [\text{mol FDCA (based on Standard 1)} + \text{mol FDCA (based on Standard 2)}] / 2$$

$$n_{[HMF]} = m_{0[HMF]}/M_{[HMF]}$$

and

$$n_{[di\text{-}HMF]} = m_{0[di\text{-}HMF]}/M_{[di\text{-}HMF]}$$

wherein $C_{[FDCA]}$ is the concentration of FDCA in % by weight in the filtrate obtained in the fourth step, $C_{0[HMF]}$ is the HMF starting concentration in % by weight, $C_{0[di\text{-}HMF]}$ is the di-HMF starting concentration in % by weight, $M_{FDCA}$, $M_{HMF}$ and $M_{di\text{-}HMF}$ are the respective molecular weights in g/mol.

The yield [mol%] for FFCA was determined *mutatis mutandis* as for the yield of FDCA.

The amount of converted HMF based on the amount of HMF and di-HMF ($C_{HMF+di\text{-}HMF}$) was calculated by the following formula:

$$C_{HMF+di\text{-}HMF} = \frac{n_{HMF}}{n_{HMF} + 2 \cdot n_{di\text{-}HMF}}$$

The minimum yield of di-HMF ($Y_{min,di\text{-}HMF}$) was calculated by:

$$\mathbf{Y}_{min,di\text{-}HMF} = \mathbf{Y}_{FDCA} - \mathbf{C}_{HMF+di\text{-}HMF}$$

In table 1, the results of the three experiments described above are shown. In all three experiments the molar amount of FDCA obtained after oxidation is larger than the molar amount of HMF provided at the beginning of the corresponding experiment. Thus, di-HMF was successfully converted into FDCA, with a considerable yield.

Moreover, table 1 shows that the yield of FDCA is increasing with increasing ratio $n_{HMF}/(n_{HMF}+2n_{di\text{-}HMF})$.

## Claims

1. Process for preparing furane-2,5-dicarboxylic acid comprising the following step:

(a) preparing or providing a starting mixture comprising

5-(hydroxymethyl)furfural (HMF),
5,5'-[oxy-bis(methylene)]bis-2-furfural (di-HMF), and
water,

wherein the total amount of water in the starting mixture is at least 50 wt-%, based on the total weight of the starting mixture and
wherein the pH of the starting mixture is in the range of from 4.0 to 7.0,
(b) subjecting said starting mixture to oxidation conditions in the presence of an oxygen-containing gas and a catalytically effective amount of a heterogeneous catalyst comprising one or more noble metals on a support so that both HMF and di-HMF react to give furane-2,5-dicarboxylic acid in a product mixture also comprising water

**2.** Process according to claim 1, wherein
the starting mixture has a molar ratio of HMF to di-HMF in the range of from 100 to 0.8, preferably in the range of from 100 to 0.9,
and/or
the total weight of HMF and di-HMF in the starting mixture is in the range of from 0.1 to 50 wt.-%, preferably in the range of from 1 to 30 wt.-%, more preferably in the range of from 1 to 10wt.-%, based on the total weight of the starting mixture.

**3.** Process according to any preceding claim, wherein the pH of the product mixture is below 7 and wherein preferably the pH of the product mixture is in the range of from 1 to 4.

**4.** Process according to any preceding claim, wherein said starting mixture at a temperature in the range of from 70°C to 200°C, preferably in the range of from 80°C to 180°C, more preferably in the range of from 90°C to 170°C, even more preferably in the range of from 100°C to 135°C, is subjected to said oxidation conditions in the presence of said oxygen-containing gas and said catalytically effective amount of a heterogeneous catalyst comprising one or more noble metals on a support, so that both HMF and di-HMF react to give furane-2,5-dicarboxylic acid in the product mixture also comprising water and oxidation by-products.

**5.** Process according to any preceding claim, wherein said starting mixture is subjected to said oxidation conditions in a pressurized reactor, wherein the oxygen partial pressure in the reactor at least temporarily is in the range of from 1 to 100 bar, preferably in the range of from 1 to 20 bar, during the reaction of both HMF and dl-HMF to furane-2,5-dicarboxylic acid.

**6.** Process according to any preceding claim, wherein the total amount of acetate ions and acetic acid in said starting mixture is below 10 wt.-%, preferably below 1 wt.-%, wherein preferably the total amount of carboxylic acid ions and carboxylic acid in the starting mixture is below 10 wt.-%, preferably below 5 wt.-%.

**7.** Process according to any preceding claim, wherein the step of preparing said starting mixture comprises

(a1) preparing or providing a material mixture comprising
one, two or more compounds selected from the group consisting of hexoses, oligosaccharides comprising hexose units, and polysaccharides comprising hexose units,
(a2) subjecting said material mixture to reaction conditions so that a mixture results comprising

HMF,
di-HMF, and
water,

(a3) optionally subjecting the mixture resulting from step (a2) to additional treatment conditions, preferably without adding a carboxylic acid and/or without adding an acidic solvent for dissolving HMF and di-HMF,

so that said starting mixture results.

**8.** Process according to any preceding claim, wherein in said heterogeneous catalyst comprising one or more noble metals on a support

(i) at least one of said noble metals is selected from the group consisting of gold, platinum, iridium, palladium, osmium, silver, rhodium and ruthenium,
and/or
(ii) said support is selected from the group consisting of carbon, metal oxides, metal halides, and metal carbides.

**9.** Process according to any preceding claim, wherein in said heterogeneous catalyst comprising one or more noble metals on a support
at least one of said noble metals is selected from the group consisting of platinum, iridium, palladium, osmium, rhodium and ruthenium, preferably platinum
and
said support is carbon.

**10.** Process according to any preceding claim, wherein in said heterogeneous catalyst comprising one or more noble metals on a support
said one or one of said more noble metals is platinum and said support is carbon,
and
the content of platinum on the support is in the range of from 0.1 to 20 wt.-%, preferably 1 to 10 wt.-%, based on the total weight of the heterogeneous catalyst comprising one or more noble metals on a support.

**11.** Process according to any preceding claim, wherein in said heterogeneous catalyst comprising one or more noble metals on a support
the molar ratio of said one or one of said more noble metals to the total amount of HMF and di-HMF is in the range of from 1:1 000 000 to 1:10, preferably in the range of from 1:10 000 to 1:10, more preferably in the range of from 1:1 000 to 1:100, preferably said one or one of said more noble metals is platinum.

**12.** Process according to any preceding claim, wherein the product mixture obtained in step (b) comprises furan-2,5-di carboxylic acid in dissolved form and wherein the product mixture obtained in step (b) preferably does not comprise furan-2,5-dicarboxylic acid in solid form.

**13.** Use of a catalyst comprising one or more noble metals on a support as an heterogeneous oxidation catalyst for accelerating in an aqueous starting mixture the conversion of both HMF and di-HMF to furane-2,5-dicarboxylic acid, wherein the pH of the starting mixture is in the range of from 4.0 to 7.0.

## Patentansprüche

**1.** Verfahren zur Herstellung von Furan-2,5-dicarbonsäure, umfassend den folgenden Schritt:

(a) Herstellen oder Bereitstellen eines Ausgangsgemischs umfassend

5-(Hydroxymethyl)furfural (HMF), 5,5'-[Oxybis(methylen)]bis-2-furfural (di-HMF) und
Wasser,

wobei die Gesamtmenge an Wasser in dem Ausgangsgemisch wenigstens 50 Gew.-%, bezogen auf das Gesamtgewicht des Ausgangsgemischs, beträgt und
wobei der pH-Wert des Ausgangsgemischs in dem Bereich von 4,0 bis 7,0 liegt,
(b) Unterwerfen des Ausgangsgemischs an Oxidationsbedingungen in Gegenwart eines sauerstoffhaltigen Gases und einer katalytisch wirksamen Menge eines heterogenen Katalysators, der ein oder mehrere Edelmetalle auf einem Träger umfasst, so dass sowohl HMF als auch di-HMF reagieren, um Furan-2,5-dicarbonsäure in einem Produktgemisch, das auch Wasser umfasst, zu ergeben.

**2.** Verfahren gemäß Anspruch 1, wobei
das Ausgangsgemisch ein Molverhältnis von HMF zu di-HMF in dem Bereich von 100 bis 0,8, vorzugsweise in dem Bereich von 100 bis 0,9, aufweist,
und/oder
das Gesamtgewicht von HMF und di-HMF in dem Ausgangsgemisch in dem Bereich von 0,1 bis 50 Gew.-%, vorzugsweise in dem Bereich von 1 bis 30 Gew.-%, bevorzugter in dem Bereich von 1 bis 10 Gew.-%, bezogen auf das Gesamtgewicht des Ausgangsgemischs, liegt.

3. Verfahren gemäß einem der vorstehenden Ansprüche, wobei der pH-Wert des Produktgemischs kleiner als 7 ist und wobei der pH-Wert des Produktgemischs vorzugsweise in dem Bereich von 1 bis 4 liegt.

4. Verfahren gemäß einem der vorstehenden Ansprüche, wobei das Ausgangsgemisch bei einer Temperatur in dem Bereich von 70 °C bis 200 °C, vorzugsweise in dem Bereich von 80 °C bis 180 °C, bevorzugter in dem Bereich von 90 °C bis 170 °C, noch bevorzugter in dem Bereich von 100 °C bis 135 °C, den Oxidationsbedingungen in Gegenwart des sauerstoffhaltigen Gases und der katalytisch wirksamen Menge eines heterogenen Katalysators, der ein oder mehrere Edelmetalle auf einem Träger umfasst, unterzogen wird, so dass sowohl HMF als auch di-HMF reagieren, um Furan-2,5-dicarbonsäure in dem Produktgemisch, das auch Wasser und Oxidationsnebenprodukte umfasst, zu ergeben.

5. Verfahren gemäß einem der vorstehenden Ansprüche, wobei das Ausgangsgemisch den Oxidationsbedingungen in einem druckbeaufschlagten Reaktor unterzogen wird, wobei der Sauerstoffpartialdruck in dem Reaktor während der Reaktion von HMF und di-HMF zu Furan-2,5-dicarbonsäure wenigstens zeitweise in dem Bereich von 1 bis 100 bar, vorzugsweise in dem Bereich von 1 bis 20 bar, liegt.

6. Verfahren gemäß einem der vorstehenden Ansprüche, wobei die Gesamtmenge an Acetat-Ionen und Essigsäure in dem Ausgangsgemisch kleiner als 10 Gew.-%, vorzugsweise kleiner als 1 Gew.-%, ist, wobei vorzugsweise die Gesamtmenge an Carbonsäure-Ionen und Carbonsäure in dem Ausgangsgemisch kleiner als 10 Gew.-%, vorzugsweise kleiner als 5 Gew.-%, ist.

7. Verfahren gemäß einem der vorstehenden Ansprüche, wobei der Schritt der Herstellung des Ausgangsgemischs umfasst:

   (a1) Herstellen oder Bereitstellen eines Materialgemischs umfassend
   eine, zwei oder drei Verbindungen ausgewählt aus der Gruppe bestehend aus Hexosen, Oligosacchariden, die Hexoseeinheiten umfassen, und Polysacchariden, die Hexoseeinheiten umfassen,
   (a2) Unterwerfen des Materialgemischs an Reaktionsbedingungen, bei denen ein Gemisch entsteht, das umfasst:

      HMF,
      di-HMF und
      Wasser,

   (a3) gegebenenfalls Unterwerfen des bei Schritt (a2) erhaltenen Gemischs an zusätzliche Behandlungsbedingungen, vorzugsweise ohne Zugeben einer Carbonsäure und/oder ohne Zugeben eines sauren Lösungsmittels zum Lösen von HMF und di-HMF,

   so dass das Ausgangsgemisch erhalten wird.

8. Verfahren gemäß einem der vorstehenden Ansprüche, wobei bei dem heterogenen Katalysator, der ein oder mehrere Edelmetalle auf einem Träger umfasst,

   (i) wenigstens eines der Edelmetalle ausgewählt ist aus der Gruppe bestehend aus Gold, Platin, Iridium, Palladium, Osmium, Silber, Rhodium und Ruthenium,
   und/oder
   (ii) der Träger ausgewählt ist aus der Gruppe bestehend aus Kohlenstoff, Metalloxiden, Metallhalogeniden und Metallcarbiden.

9. Verfahren gemäß einem der vorstehenden Ansprüche, wobei bei dem heterogenen Katalysator, der ein oder mehrere Edelmetalle auf einem Träger umfasst, wenigstens eines der Edelmetalle ausgewählt ist aus der Gruppe bestehend aus Platin, Iridium, Palladium, Osmium, Rhodium und Ruthenium, vorzugsweise Platin, und
   der Träger Kohlenstoff ist.

10. Verfahren gemäß einem der vorstehenden Ansprüche, wobei bei dem heterogenen Katalysator, der ein oder mehrere Edelmetalle auf einem Träger umfasst, das eine oder eines der mehreren Edelmetalle Platin ist und der Träger Kohlenstoff ist
   und

der Gehalt an Platin auf dem Träger in dem Bereich von 0,1 bis 20 Gew.-%, vorzugsweise 1 bis 10 Gew.-%, bezogen auf das Gesamtgewicht des heterogenen Katalysators, der ein oder mehrere Edelmetalle auf einem Träger umfasst, liegt.

**11.** Verfahren gemäß einem der vorstehenden Ansprüche, wobei bei dem heterogenen Katalysator, der ein oder mehrere Edelmetalle auf einem Träger umfasst,
das Molverhältnis des einen oder eines der mehreren Edelmetalle zu der Gesamtmenge an HMF und di-HMF in dem Bereich von 1:1.000.000 bis 1:10, vorzugsweise in dem Bereich von 1:10.000 bis 1:10, bevorzugter in dem Bereich von 1:1.000 bis 1:100, liegt, wobei das eine oder eines der mehreren Edelmetalle vorzugsweise Platin ist.

**12.** Verfahren gemäß einem der vorstehenden Ansprüche, wobei das bei Schritt (b) erhaltene Produktgemisch Furan-2,5-dicarbonsäure in gelöster Form umfasst und wobei das bei Schritt (b) erhaltene Produktgemisch vorzugsweise keine Furan-2,5-dicarbonsäure in fester Form umfasst.

**13.** Verwendung eines Katalysators, der ein oder mehrere Edelmetalle auf einem Träger umfasst, als heterogener Oxidationskatalysator zum Beschleunigen der Umwandlung von sowohl HMF als auch di-HMF zu Furan-2,5-dicarbonsäure in einem wässrigen Ausgangsgemisch, wobei der pH-Wert des Ausgangsgemischs in dem Bereich von 4,0 bis 7,0 liegt.

**Revendications**

**1.** Procédé pour la préparation de l'acide furanne-2,5-dicarboxylique comprenant l'étape suivante :

(a) préparation ou mise à disposition d'un mélange de départ comprenant
du 5-(hydroxyméthyl)furfural (HMF),
du 5,5'-[oxy-bis(méthylène)]bis-2-furfural (di-HMF), et
de l'eau,
la quantité totale d'eau dans le mélange de départ étant d'au moins 50 % en poids, sur la base du poids total du mélange de départ et le pH du mélange de départ se situant dans la plage de 4,0 à 7,0,
(b) soumission dudit mélange de départ à des conditions d'oxydation en présence d'un gaz contenant de l'oxygène et d'une quantité catalytiquement efficace d'un catalyseur hétérogène comprenant un ou plusieurs métaux nobles sur un support de sorte qu'à la fois le HMF et le di-HMF réagissent pour donner de l'acide furanne-2,5-dicarboxylique dans un mélange de produits comprenant aussi de l'eau.

**2.** Procédé selon la revendication 1, le mélange de départ possédant un rapport molaire de HMF à di-HMF dans la plage de 100 à 0,8, préférablement dans la plage de 100 à 0,9,
et/ou
le poids total de HMF et de di-HMF dans le mélange de départ se situant dans la plage de 0,1 à 50 % en poids, préférablement dans la plage de 1 à 30 % en poids, plus préférablement dans la plage de 1 à 10 % en poids, sur la base du poids total du mélange de départ.

**3.** Procédé selon l'une quelconque des revendications précédentes, le pH du mélange de produits étant inférieur à 7 et préférablement le pH du mélange de produits se situant dans la plage de 1 à 4.

**4.** Procédé selon l'une quelconque des revendications précédentes, ledit mélange de départ à une température dans la plage de 70 °C à 200 °C, préférablement dans la plage de 80 °C à 180 °C, plus préférablement dans la plage de 90 °C à 170 °C, encore plus préférablement dans la plage de 100 °C à 135 °C, étant soumis auxdites conditions d'oxydation en présence dudit gaz contenant de l'oxygène et de ladite quantité catalytiquement efficace d'un catalyseur hétérogène comprenant un ou plusieurs métaux nobles sur un support, de sorte qu'à la fois le HMF et le di-HMF réagissent pour donner de l'acide furanne-2,5-dicarboxylique dans le mélange de produits comprenant aussi de l'eau et des sous-produits d'oxydation.

**5.** Procédé selon l'une quelconque des revendications précédentes, ledit mélange de départ étant soumis auxdites conditions d'oxydation dans un réacteur pressurisé, la pression partielle d'oxygène dans le réacteur étant au moins temporairement dans la plage de 1 à 100 bar(s), préférablement dans la plage de 1 à 20 bar(s), pendant la réaction à la fois de HMF et de di-HMF en acide furanne-2,5-dicarboxylique.

6. Procédé selon l'une quelconque des revendications précédentes, la quantité totale d'ions acétate et d'acide acétique dans ledit mélange de départ étant inférieure à 10 % en poids, préférablement inférieure à 1 % en poids, préférablement la quantité totale d'ions d'acide carboxylique et d'acide carboxylique dans le mélange de départ étant inférieure à 10 % en poids, préférablement inférieure à 5 % en poids.

7. Procédé selon l'une quelconque des revendications précédentes, l'étape de préparation dudit mélange de départ comprenant

(a1) la préparation ou la mise à disposition d'un mélange de matières comprenant
un, deux ou plusieurs composés choisis dans le groupe constitué par les hexoses, les oligosaccharides comprenant des motifs hexose, et des polysaccharides comprenant des motifs hexose,
(a2) la soumission dudit mélange de matières à des conditions de réaction de sorte qu'un mélange en résulte, comprenant
du HMF,
du di-HMF, et
de l'eau,
(a3) éventuellement la soumission du mélange résultant de l'étape (a2) à des conditions de traitement supplémentaires, préférablement sans ajout d'un acide carboxylique et/ou sans ajout d'un solvant acide pour la dissolution du HMF et du di-HMF,

de sorte que ledit mélange de départ en résulte.

8. Procédé selon l'une quelconque des revendications précédentes, dans lequel dans ledit catalyseur hétérogène comprenant un ou plusieurs métaux nobles sur un support

(i) au moins un desdits métaux nobles est choisi dans le groupe constitué par l'or, le platine, l'iridium, le palladium, l'osmium, l'argent, le rhodium et le ruthénium,
et/ou
(ii) ledit support est choisi dans le groupe constitué par le carbone, les oxydes métalliques, les halogénures métalliques, et les carbures métalliques.

9. Procédé selon l'une quelconque des revendications précédentes, dans lequel dans ledit catalyseur hétérogène comprenant un ou plusieurs métaux nobles sur un support
au moins un desdits métaux nobles est choisi dans le groupe constitué par le platine, l'iridium, le palladium, l'osmium, le rhodium et le ruthénium, préférablement le platine
et
ledit support est le carbone.

10. Procédé selon l'une quelconque des revendications précédentes, dans lequel dans ledit catalyseur hétérogène comprenant un ou plusieurs métaux nobles sur un support
ledit métal noble ou l'un desdits métaux nobles est le platine et ledit support est le carbone
et
la teneur en platine sur le support se situe dans la plage de 0,1 à 20 % en poids, préférablement de 1 à 10 % en poids, sur la base du poids total du catalyseur hétérogène comprenant un ou plusieurs métaux nobles sur un support.

11. Procédé selon l'une quelconque des revendications précédentes, dans lequel dans ledit catalyseur hétérogène comprenant un ou plusieurs métaux nobles sur un support
le rapport molaire dudit métal noble ou d'un desdits métaux nobles à la quantité totale de HMF et de di-HMF se situe dans la plage de 1:1.000.000 à 1:10, préférablement dans la plage de 1:10.000 à 1:10, plus préférablement dans la plage de 1:1000 à 1:100, préférablement ledit métal noble ou l'un desdits métaux nobles est le platine.

12. Procédé selon l'une quelconque des revendications précédentes, le mélange de produits obtenu dans l'étape (b) comprenant de l'acide furanne-2,5-dicarboxylique sous forme dissoute et le mélange de produits obtenu dans l'étape (b) préférablement ne comprenant pas d'acide furanne-2,5-dicarboxylique sous forme solide.

13. Utilisation d'un catalyseur comprenant un ou plusieurs métaux nobles sur un support en tant qu'un catalyseur d'oxydation hétérogène pour l'accélération, dans un mélange de départ aqueux, de la conversion à la fois de HMF et de di-HMF en acide furanne-2,5-dicarboxylique,

le pH du mélange de départ se situant dans la plage de 4,0 à 7,0.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2994645 A
- WO 2008054804 A2
- WO 2013033081 A2
- US 2008103318 A
- WO 2012017052 A1
- EP 0356703 A2
- WO 2013191944 A1
- WO 2013033081 A


### Non-patent literature cited in the description


- Carbohydrates as Organic Raw Materials. **LICHTENTHALER, F.W.** Ullmann's Encyclopedia of Industrial Chemistry. Wiley-VCH Verlag GmbH & Co. KGaA, 2010
- **HICHAM AIT RASS et al.** selective aqueous phase oxidation of 5-hydroxymethyl furfural to 2,5-furandicarboxylic acid over Pt/C catalysts. *GREEN CHEMISTRY,* 01 January 2013, vol. 15 (8), 2240
- **J. MA ; Y. PANG ; M. WANG ; J. XU ; H. MA ; X. NIE.** *J. Mater. Chem.,* 2012, vol. 22, 3457-3461
- **A. J. CARPENTER ; D. J. CHADWICK.** *Tetrahedron,* 1985, vol. 41 (18), 3803-3812